# EUROPÄISCHE PATENTSCHRIFT

(11) **EP 2 954 442 B1**
(45) Veröffentlichungstag und Bekanntmachung des Hinweises auf die Patenterteilung: **22.07.2020**
(21) Anmeldenummer: 14702453.3
(22) Anmeldetag: 31.01.2014
(51) Int. Cl.: A61M 1/16, G16H 10/60, G16H 40/63

(54) **VORRICHTUNG UND VERFAHREN ZUR ERZEUGUNG UND ANZEIGE VON FÜR MEDIZINISCHE GERÄTE UND MEDIZINISCHE BEHANDLUNGEN SPEZIFISCHEN GRAFIKKODIERUNGEN**
DEVICE AND METHOD FOR GENERATING AND DISPLAYING GRAPHIC CODES SPECIFIC TO MEDICAL APPARATUS AND MEDICAL TREATMENTS
DISPOSITIF ET PROCÉDÉ PERMETTANT LA PRODUCTION ET L'AFFICHAGE DE CODAGES GRAPHIQUES SPÉCIFIQUES À DES APPAREILS MÉDICAUX ET À DES TRAITEMENTS MÉDICAUX

(30) Priorität: 11.02.2013 DE 102013002231; 11.02.2013 US 201361763303 P
(43) Veröffentlichungstag der Anmeldung: 16.12.2015
(73) Patentinhaber: Fresenius Medical Care Deutschland GmbH, 61352 Bad Homburg (DE)
(72) Erfinder: BLÜMLER, Holger, 61381 Friedrichsdorf (DE); KLEWINGHAUS, Jürgen, 61440 Oberursel (DE); ADAM, Pascal, 60435 Frankfurt (DE); LOTZ, Bastian, 35394 Giessen (DE); PAETZOLD, Ingmar, 63743 Aschaffenburg (DE); COLLINI, Danilo, Milano (IT)
(74) Vertreter: Dreyhsig, Jörg
(86) Internationale Anmeldenummer: PCT/EP2014/000262
(87) Internationale Veröffentlichungsnummer: WO 2014/121909

(56) Entgegenhaltungen:
- WO-A2-03/026558
- DE-A1-102011 011 767
- GB-A- 2 459 686
- US-A1- 2006 206 356
- US-A1- 2008 149 701
- US-A1- 2012 067 943
- US-A1- 2012 185 267

## Beschreibung

### Technisches Gebiet

Die Erfindung betrifft das Gebiet der Erzeugung und Anzeige von Grafikkodierungen, die auf ein medizinisches Fluidmanagementgerät und/oder auf eine mit dem medizinischen Fluidmanagementgerät durchgeführte Behandlung, bezogen sind.

### Hintergrund

Unter medizinischen Fluidmanagementgeräten werden hierbei insbesondere Geräte zur Leitung, Behandlung und/oder Verteilung von Flüssigkeiten und/oder Gasen verstanden, bei denen über eine Fluidleitung Fluid zwischen einem Patienten und einer Fluidbehandlungskomponente und/oder einer Fluidquelle transportiert werden.

Unter Fluidmanagementgeräten werden insbesondere auch Fluidbehandlungsgeräte wie Blutbehandlungsgeräte verstanden, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin, deren Inhalt hiermit vollumfänglich im Offenbarungsgehalt der vorliegenden Anmeldung einbezogen wird.

Die Dialyse ist ein Verfahren zur Blutreinigung von Patienten mit akuter oder chronischer Niereninsuffizienz. Grundsätzlich unterscheidet man hierbei zwischen Verfahren mit einem extrakorporalen Blutkreislauf, wie der Hämodialyse, der Hämofiltration oder der Hämodiafiltration und der Peritonealdialyse, die keinen extrakorporalen Blutkreislauf aufweist.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von Plasmawasser durch einen Druckgradienten an der Membran des Dialysators.

Die Verfahren der Hämodialyse, der Hämofiltration und der Hämodiafiltration werden in der Regel mit automatischen Hämodialysegeräten durchgeführt, wie sie beispielsweise von der Anmelderin unter der Bezeichnung 5008 vertrieben werden.

Die Plasmapherese ist ein Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben.

Bei der Peritonealdialyse wird die Bauchhöhle eines Patienten über einen durch die Bauchdecke geführten Katheter mit einer Dialyseflüssigkeit befüllt, die ein Konzentrationsgefälle gegenüber den körpereigenen Flüssigkeiten aufweist. Über das als Membran wirkende Bauchfell (Peritoneum) treten die im Körper vorliegenden Giftstoffe in die Bauchhöhle über. Nach einigen Stunden wird die sich in der Bauchhöhle des Patienten befindliche, nunmehr verbrauchte Dialyse-flüssigkeit ausgetauscht. Durch osmotische Vorgänge kann Wasser aus dem Blut des Patienten über das Bauchfell in die Dialyseflüssigkeit übertreten und den Patienten somit entwässern.

Das Verfahren zur Peritonealdialyse wird in der Regel mit Hilfe von automatischen Peritonealdialysegeräten, wie sie beispielsweise von der Anmelderin unter der Bezeichnung sleep.safe vertrieben werden, durchgeführt.

Dialysegeräte, als Beispiel für komplexe medizinische Fluidmanagementgeräte, haben umfangreiche Funktionen. Um diese Funktionen zu steuern, sind medizinische Fluidmanagementgeräte wie Dialysegeräte mit zumindest einer Steuervorrichtung ausgerüstet. Diese können als CPU (central processing unit) oder Mikrokontroller ausgeführt sein, die von Softwareprogrammen programmiert werden. Die Bedienung solcher Geräte geschieht häufig über Touchscreendisplays. Ein solches Touchscreendisplay kombiniert in einer gemeinsamen Oberfläche eine Ein- und eine Ausgabevorrichtung, in dem es eine berührungsempfindliche Oberfläche bereitstellt, mit der Bedienereingaben detektierbar sind.

Mögliche alternative Ausführungen sehen die räumliche Trennung von Ein- und Ausgabevorrichtung vor, beispielsweise durch ein konventionelles Display, beispielsweise als CRT-Monitor (Cathode Ray Tube), LCD (Liquid Christal Display), Plasma- oder OLED-Display (Organic Light Emitting Device) ausgeführt, als Ausgabevorrichtung und einem davon räumlich getrennten Touchpad, das eine berührempfindliche Oberfläche bereitstellt, mit der Bedienereingaben detektierbar sind, als Eingabevorrichtung.

Im Folgenden sollen Ausführungsformen eines medizinischen Fluidmanagementgeräts anhand eines Dialysegeräts beschrieben werden. Beispiele hierfür sind Infusionsgeräte für medizinische Flüssigkeiten, Herz-Kreislaufunterstützungsgeräte mit extrakorporalem Blutkreislauf, Leberunterstützungsgeräte mit extrakorporalem Blutkreislauf oder Ähnliches. Jede Ausführungsform, die im Folgenden am Beispiel eines Dialysegeräts als Beispiel für ein medizinisches Fluidmanagementgerät beschrieben wird, kann ohne weiteres auf ein anderes medizinisches Fluidmanagementgerät übertragen werden. Derartige Ausführungsformen werden explizit von den beigefügten Ansprüchen umfasst.

An Niereninsuffizienz leidende Patienten müssen sich regelmäßigen Dialyse-behandlungen unterziehen. Diese Behandlungen erfolgen im Falle der Hämodialyse oder generell bei Verfahren, bei denen das Blut in einem extrakorporalen Blutkreislauf gereinigt wird, zumeist unter der Aufsicht von medizinischem Personal in einer Dialysestation.

Solche Dialysestationen umfassen innerhalb eines Raumes bzw. eines Gebäudes mehrere Dialysegeräte, an denen gleichzeitig eine Vielzahl von Patienten behandelt werden können.

Es ist für das betreuende medizinische Personal von Bedeutung, dass die Identität des behandelten Patienten bekannt ist und eindeutig einem bestimmten Dialysegerät zugeordnet ist. Nur so kann gewährleistet werden, dass der konkrete Patient die für ihn vorgesehene Behandlung erhält.

Zu diesem Zweck sind patientenindividuelle Patientenkarten bekannt. Derartige Patientenkarten umfassen zumindest eine Schnittstelle zur Kommunikation mit einer entsprechenden Schnittstelle an dem Dialysegerät und zumindest einen Speicher, in dem individuelle Daten des Patienten abgelegt werden können.

Die individuellen Daten des Patienten können den Namen des Patienten umfassen. Zusätzlich lassen sich auch physiologische Daten, wie Körpergröße, Alter, Geschlecht, Normalblutdruck usw. abspeichern, sowie Daten vergangener Behandlungen, wie Datum oder Dauer der Behandlung sowie Einstellungen des betreffenden Geräts, wie beispielsweise Blutflussrate, Dialysatflussrate, verwendete Lösungen und Filter usw.

Zu Beginn der Behandlung macht sich der Patient beispielsweise durch Einlesen der Patientenkarte an dem Dialysegerät bekannt. Während der Behandlung kann der Name des Patienten auf dem Bildschirm des Geräts angezeigt werden.

Das medizinische Personal erfasst somit auf einen Blick die Identität des Patienten. Nachteilig erweist sich, dass alleine die Kenntnis des Namens des Patienten i.d.R. noch keine Beurteilung der medizinischen Situation des Patienten erlaubt. Hierzu bedarf es der Kenntnis aller mit dem Patienten verknüpften physiologischen und behandlungsbezogenen Daten.

Diese werden in der Regel nicht am Bildschirm des Dialysegeräts direkt angezeigt. Darüber hinaus sind auch medizinische Fluidmanagementgeräte bekannt, die den Namen des Patienten nicht anzeigen.

Des Weiteren sind auch andere Information, die das Dialysegerät selbst betreffen, oder eine damit durchgeführte Behandlung oder auch die Gesamtsituation innerhalb einer Dialysestation wichtig und müssen bisher auf vielfältige Weise den Personen bekannt gemacht werden, die als Empfänger der Information bestimmt sind.

Solche spezifischen Daten können beispielsweise umfassen: den Typ, das Baujahr und die Laufzeit des Geräts, Werbeinformationen über das Gerät, Informationen über den Wartungsstatus des Geräts, Informationen über die Ausrüstung des Geräts bzw. über die an dem Gerät befindlichen Zubehörteile und gegebenenfalls auch Fehlermeldungen. Darüber hinaus kann es auch wichtig sein, Informationen über die Belegung einer Dialysestation zu erhalten, also welche Personen zur Zeit gerade an welchen Dialysegeräten behandelt werden, wie deren Behandlungsstatus momentan gerade ist, beispielsweise wie lange die Dialyse für jeden aktuell behandelten Patienten noch ansteht, oder welche Patienten innerhalb eines bestimmten Zeitintervalls in der Zukunft für eine Behandlung vorgesehen sind.

Die Information, die übermittelt werden soll, richtet sich je nach Informationsinhalt an unterschiedliche Personen. So können unterschiedliche Personen unterschiedliche Berechtigungen haben, um Informationen, die vom Dialysegerät ausgesendet werden, zu empfangen oder nicht. Beispielsweise sind Informationen, die den Wartungszustand oder Fehlermeldungen des Geräts betreffen, an technisches Personal gerichtet, während medizinische Daten an medizinisches Personal gesendet und auch nur von diesem gelesen werden soll.

Weiterhin können allgemeine Informationen, wie beispielsweise Werbebotschaften, an eine dritte Gruppe gerichtet sein, beispielsweise an Besucher einer Fachmesse.

Der Bediener eines Dialysegeräts, insbesondere der behandelnde Arzt, kann für die Anzeige der für ihn bestimmten Information ein Gerät mitführen, dem die Daten des entsprechenden Patienten übermittelt werden, oder das eine Vorrichtung zum Einlesen der Patientenkarte vorhält, um sich einen Überblick über die medizinische Situation zu verschaffen.

Derartige Geräte können eigens dafür konstruierte Geräte sein, die in der Regel transportabel sind, oder handelsübliche mobile Computer, wie SmartPhones oder Tablet-PCs, die die entsprechenden Daten über eine Datenschnittstelle erhalten. Diese Datenschnittstelle basiert in einer Ausführungsform auf drahtlose Kommunikation wie Bluetooth, WLAN oder Mobilfunk-Technologie.

In der Druckschrift US2012/0185267 A1 ist ein Patientenbehandlungssystem mit mehreren Komponenten erläutert, welche miteinander kommunizieren.

### Beschreibung

Es ist daher Aufgabe der vorliegenden Erfindung, eine Möglichkeit zu schaffen, die Zuordnung von einem Patient zu einem Dialysegerät herzustellen und darüber hinaus eine verbesserte Übermittlung von physiologischen bzw. behandlungsbezogenen Daten und/oder gerätespezifischen Daten zu ermöglichen.

Die Lösung dieser Aufgaben erfolgt erfindungsgemäß durch ein Verfahren mit den Merkmalen des Anspruch 1 und durch ein System mit den Merkmalen des Anspruchs 6.

Vorteilhafte Ausführungsformen sind Gegenstand der Unteransprüche.

Unter Informationen, die das medizinische Fluidmanagementgerät betreffen, sind nach der Lehre der vorliegenden Offenbarung sowohl behandlungsbezogene Daten, insbesondere aufgenommene Messwerte wie Blutflussrate, Dialysatflussrate oder auch über die Behandlung akkumulierte Volumina wie Ultrafiltrationsvolumen oder Substituatsvolumen, sowie auch Verläufe über eine oder mehrere Behandlungen dieser beispielhaften Werte, als auch Daten, die die Ausstattung bzw. Aufrüstung und den Zustand des medizinischen Geräts betreffen, zu verstehen. Hierzu zählen beispielsweise Geräte- bzw. Maschinenidentifikationsnummern, Netzwerkadressen, Mac-Adressen, Softwareversionsnummern, Laufzeit der Maschine, Standort des Geräts, Fehlermeldungen, eingebautes Zubehör, angeschlossenes Zubehör wie Schlauchsets, Beutel, Filter, Tropfkammern und Ähnliches, sowie Werbe- oder allgemeine Informationen, die das Fluidmanagementgerät betreffen.

Medizinische Fluidmanagementgeräte, wie beispielsweise Dialysegeräte, verfügen oftmals über mindestens eine Steuervorrichtung. Eine solche Steuervorrichtung steuert die Komponenten des medizinischen Geräts unter Anderem anhand derer ihr bekannt gemachten das medizinische Gerät kennzeichnenden Parameterwerte. Solche Steuervorrichtungen sind häufig mit programmierbaren Mikroprozessoren oder Mikrocontrollern ausgerüstet, wobei die sie steuernden Programme in dafür vorgesehenen Programmdatenspeichern abgelegt sind. Eine Steuervorrichtung kann auf einem oder mehreren Prozessoren implementiert sein. So sind oftmals auch mehrere Steuervorrichtungen in einem medizinischen Gerät vorgehalten. Im Sinne der vorliegenden Offenbarung wird unter dem Begriff Steuervorrichtung auch eine Vielzahl von Steuervorrichtungen verstanden.

Weiterhin verfügen medizinische Geräte, wie beispielsweise Dialysegeräte, oftmals über Sende- und Empfangsvorrichtungen, wobei unter Sende- und Empfangsvorrichtung alle Vorrichtungen verstanden werden, mit denen Daten, insbesondere digitale Daten zu einem entfernten Gerät gesendet bzw. von einem entfernten Gerät empfangen werden können. Hierzu zählen insbesondere Datenschnittstellen, wie Netzwerkschnittstellen, die sowohl kabelgebunden (beispielsweise RJ45) als auch nicht kabelgebunden (beispielsweise WLAN, Bluetooth, Infrarot oder UMTS) sein können.

Weiterhin können medizinische Geräte auch über Einlesevorrichtungen zum Einlesen grafischer Informationen, wie Bilder oder Strichcodes umfassen. Solche Einlesevorrichtungen können Scanner oder Kameras sein.

Ein Patient, der sich einer Dialysebehandlung unterzieht, leidet häufig an chronischer Niereninsuffizienz. Das bedeutet, dass er sich regelmäßigen Behandlungen unterziehen muss, die pro Behandlung mehrere Stunden dauern können.

Während der Dialysebehandlung wird das Blut des Patienten gereinigt und überschüssiges Wasser aus dem Blut des Patienten entfernt. Der Erfolg der Behandlung hängt von vielfältigen Faktoren ab und wird in der Regel vom Dialysegerät protokolliert. So können physiologische Daten, wie beispielsweise der arterielle und venöse Blutdruck im extrakorporalen Blutkreislauf oder die Bluttemperatur und andere die Behandlung betreffende Werte, wie beispielsweise die Flussraten von Blut und Dialysierflüssigkeit, sowie die Ultrafiltrations- bzw. Substitutionsrate und akkumulierte Volumina dieser Fluide über die Zeit aufgenommen und als Datensatz hinterlegt werden.

Bei der Behandlung kann dem Dialysegerät die Identität des Patienten bekannt gemacht werden. Dies kann beispielsweise dadurch geschehen, in dem der Patient eine individuelle Patientenkarte vorhält, die mit einer entsprechenden Einlesevorrichtung am Dialysegerät eingelesen werden kann.

Derartige Patientenkarten sind sogenannte Smart-Cards, die zumindest über eine Speichervorrichtung und eine Schnittstelle verfügen. In die Speichervorrichtung können Patientendaten abgespeichert werden, wie beispielsweise der Patientenname, physiologische Daten, wie Körpergröße, Alter, Geschlecht und das sogenannte Trockengewicht.

Das Trockengewicht ist dasjenige Körpergewicht eines Patienten, welches er in der Regel nach einer Dialysebehandlung haben sollte. Aufgrund der Niereninsuffizienz können dialysepflichtige Patienten häufig nicht genug oder gar kein Wasser ausscheiden. In Folge der lebensnotwendigen Wasseraufnahme über die Nahrung reichert der Patient also Wasser im Blut und im Körpergewebe an, welches durch den Ultrafiltrationsvorgang bei der Dialyse entfernt werden soll. Hierbei soll in der Regel jenes Volumen entfernt werden, das sich aus der Differenz zwischen aktuellem Körpergewicht und hinterlegtem Trockengewicht ergibt.

Weiterhin können auch Daten vergangener Behandlungen auf der Patientenkarte abgespeichert werden, beispielsweise das Datum der letzten Behandlung, die Dauer, verwendete behandlungsbestimmende Vorrichtungen, wie Filter, Lösungen, therapeutische Daten wie Ultrafiltrationsvolumen, Blutflussraten und begleitenden physiologische Messwerte wie Blutdruck, Körpertemperatur usw. Diese Daten können dann bei jeder folgenden Behandlung durch das Beschreiben des internen Speichers aktualisiert werden.

Die Schnittstelle der Patientenkarte kann vielfältig ausgeführt sein, beispielsweise als Magnetstreifen oder Kontaktflächen, die eine elektrisch leitende Verbindung mit einem Lesegerät eingehen können. Auch können Patientenkarten mit berührungslosen Schnittstellen, wie RF-ID Systemen ausgestattet sein. Um an möglichst vielen verschiedenen Geräten les- und/oder beschreibbar zu sein, können Patientenkarten auch mit einer Vielzahl verschiedener Schnittstellen ausgerüstet sein, die alternativ oder auch gleichzeitig verwendbar sein können.

Neben einem Speicher verfügen Patientenkarten häufig auch über zumindest eine Steuervorrichtung, die in der Regel als Mikroprozessor bzw. Mikrokontroller ausgeführt ist und zur Interaktion mit dafür vorgesehenen Geräten entsprechend softwareprogrammiert ist. Diese Programmierung kann im internen Speicher abgelegt sein. Die Programmierung kann durch Einspeichern einer neuen Version auch verändert bzw. aktualisiert werden.

Die Patientenkarte kann auch über eine Vielzahl von Steuervorrichtungen verfügen.

Der Patient kann sich auch in anderer Weise bei dem Dialysegerät bekannt machen. Hierbei kann auf jede Methode zurückgegriffen werden, die eine eindeutige Identifizierung des Patienten ermöglicht.

Beispielsweise kann der Patientenname auch manuell über eine Bedienereingabe am Dialysegerät eingegeben werden, beispielsweise über eine virtuelle Tastatur, die auf einem Touchscreen dargestellt wird.

Um Verwechslungen zu vermeiden, kann eine derartige Eingabe des Patientennamens durch die zusätzliche Eingabe eines Passworts durch den Patienten selbst abgesichert werden.

Eine weitere Möglichkeit stellt das Einlesen biometrischer Daten des Patienten dar, um diesen eindeutig zu identifizieren. Solche biometrische Daten können beispielsweise das Gesichtsbild des Patienten umfassen, oder aber auch dessen Fingerabdruck. Zur Erfassung derartiger Merkmale können im Dialysegerät entsprechende Vorrichtungen vorgehalten werden, beispielsweise Kameras oder Fingerabdruckscanner.

Um den Patienten nach Einlesen derartiger biometrischer Daten eindeutig zu identifizieren, ist es notwendig, dass eine Verknüpfung zwischen erfassten biometrischen Daten und Patientenidentität hinterlegt ist. Diese Hinterlegung kann im Dialysegerät selbst vorhanden sein, beispielsweise durch einen in einem internen Speicher des Dialysegeräts hinterlegten Datensatz, oder vorteilhaft in einem entfernten Gerät, beispielsweise einem Servercomputer, der mit dem Dialysegerät über eine Datenfernübertragung kommunizieren kann.

Eine weitere Möglichkeit, den Patienten eindeutig zu identifizieren, ist die Vorhaltung einer grafischen Kodierung durch den Patienten, die patientenindividuell erstellt wird. Eine solche patientenindividuelle grafische Kodierung kann beispielsweise ein Strichcode (Barcode) oder ein QR-Code (quick response code) sein, der komplexer ist, als ein konventioneller Strichcode und folglich umfangreichere Kodierungsmöglichkeiten bietet. Der QR-Code ist ein zweidimensionaler Code. Weitere zweidimensionale Codes sind der Micro-QR-Code, der Secure-QR-Code (SQRC) und der iQR-Code. Im Einklang mit der Lehre der vorliegenden Erfindung sind aber jegliche grafische Kodierungen möglich, die individuell einem Patienten zugeordnet werden können.

Um den Patienten zu identifizieren wird in einer Ausführungsform die grafische Kodierung, die der Patient vorhält, im Dialysegerät eingelesen. Die grafische Kodierung kann beispielsweise auch auf einer Patientenkarte vorhanden sein. Das Dialysegerät kann zum Einlesen der grafischen Kodierung beispielsweise eine dafür vorgesehene Kamera verwenden, oder einen Barcodescanner.

Die grafische Kodierung kann den Namen des Patienten grafisch kodiert enthalten, oder ein sonstiges Identifikationsmerkmal, beispielsweise eine Patientennummer, die mit der Identität des Patienten verknüpft ist. Die grafische Kodierung kann aber auch selbst individuell für die Identität des Patienten sein. In dem Fall wird die eingelesene grafische Kodierung mit hinterlegten Kodierungen verglichen, wobei die hinterlegten Kodierungen mit jeweils individuellen Patientendaten verknüpft sind. Auf diese Weise kann die eingelesene grafische Kodierung den Daten eines bestimmten Patienten eindeutig zugeordnet werden.

Unabhängig davon, auf welche Weise dem Dialysegerät die Identität des Patienten bekannt gemacht wird, ist im Einklang mit der Lehre der vorliegenden Erfindung vorgesehen, dass eine eindeutige Zuordnung der Patientenidentität mit dem jeweiligen Dialysegerät erfolgt.

Um eine eindeutige Zuordnung zwischen Patient und Dialysegerät zu generieren, ist weiterhin vorgesehen, dass auch das Dialysegerät über eindeutige Identifikationsmerkmale verfügt. Hierzu geeignet sind individuelle Maschinennummern oder feste IP-Adressen, unter denen Dialysegeräte in einem Datennetz kommunizieren oder MAC-Adressen (Media-Access-Control-Adressen), die die Hardware von Dialysegeräten eindeutig identifizieren.

Derartige Identifikationsmerkmale sind in der Regel in dem Dialysegerät vorgehalten und können über Datenfernübertragung an ein entferntes Gerät, beispielsweise an einen Servercomputer übermittelt werden.

Macht sich also ein Patient in einer der oben beschriebenen Weisen am Dialysegerät bekannt, können so sowohl die Patientenidentität bzw. ein mit der Patientenidentität verknüpfter Datensatz als auch die Identität des Dialysegeräts an ein entferntes Gerät, beispielsweise einen Servercomputer gesendet werden. Beide Identitäten sind sowohl im Dialysegerät als auch im Servercomputer miteinander verknüpft. Auf diese Weise findet eine Paarung dieser individuellen Daten statt.

Eine Paarung der Patientenidentität mit der Identität des Dialysegeräts kann auch erfolgen, in dem die Patientenidentität und das Identifikationsmerkmal des Dialysegeräts an einem mobilen Computer eingelesen werden und diese Daten vom mobilen Computer an einen entfernten Computer, beispielsweise einen Servercomputer gesendet werden, wo sie miteinander verknüpft werden. Dies ist dann von Vorteil, wenn das Dialysegerät über keine Datenverbindung zum entfernten Computer verfügt, oder keine Möglichkeit bietet, Patientenidentitäten einzulesen, weil es über keine Datenschnittstelle verfügt und/oder keine Eingabemöglichkeit für die Patientenidentität bietet, bzw. kein Lesegerät für eine Patientenkarte aufweist.

Das Einlesen der Patientenidentität am mobilen Computer kann beispielsweise dadurch erfolgen, in dem eine patientenindividuelle Grafikkodierung, die der Patient mit sich führt, eingelesen wird. Der mobile Computer kann zu diesem Zweck aber auch mit Mitteln zum Einlesen einer Patientenkarte ausgestattet sein.

Im Servercomputer können neben den patientenindividuellen Daten auch Daten hinterlegt sein, die mit der Identität des Dialysegeräts verknüpft sind.

Derartige Daten können umfassen: Standort des Dialysegeräts, Besitzer des Dialysegeräts, Auskunft über Ausrüstung des Geräts, Status der Wartung des Geräts, Meldungen über Fehlfunktionen, die vom Dialysegerät an den Servercomputer gesendet werde können, Betriebszeit des Geräts usw.

In einer Dialysestation sind in der Regel mehrere Dialysegeräte vorhanden, an denen zur gleichen Zeit eine Vielzahl von Patienten behandelt wird. Im Einklang mit der Lehre der vorliegenden Erfindung können sich alle Patienten auf einer der oben beschriebenen Weisen an den jeweiligen Dialysegeräten bekannt machen, und alle Dialysegräte können sowohl die Patientenidentifikationsmerkmale als auch die Dialysegeräteidentifikationsmerkmale an einen entfernten Servercomputer senden.

Durch die Hinterlegung dieser Daten in einem Servercomputer werden in diesem Informationen, die die aktuelle Belegung der Dialysestation, gegebenenfalls mit allen relevanten medizinischen, therapeutischen und gerätebezogenen Daten, betreffen, vorgehalten. Diese Informationen können im Einklang mit der Lehre der vorliegenden Erfindung an weitere Geräte versendet werden.

Ein solches Gerät kann beispielsweise vom betreuenden medizinischen oder vom technischen Personal vorgehalten werden.

Ein solches Gerät kann beispielsweise ein mobiles Endgerät, etwa ein Smartphone oder ein Tablet-PC sein und wird im Folgenden als externer Computer bezeichnet.

Derartige externe Computer verfügen in der Regel neben Schnittstellen zur Datenkommunikation über Kameras, die dazu verwendet werden können, grafische Kodierungen, im Folgenden auch Grafikkodierungen genannt, einzulesen. Die eingelesenen grafischen Kodierungen können im externen Computer dekodiert werden. Hierzu verwendet der externe Computer ein spezielles Computerprogramm. Derartige Computerprogramme werden insbesondere bei Smartphones oder Tablet-PCs Applikationen oder kurz App genannt.

Eine solche grafische Kodierung kann der Patient in oben beschriebener Weise vorhalten. Darüber hinaus kann auch das Dialysegerät eine solche Kodierung aufweisen, beispielsweise als Aufkleber ausgeführt.

Die Ausführungsform als Aufkleber hat den Vorteil, dass dieser einfach herstellbar und applizierbar ist. Ein solcher Aufkleber kann an einer beliebigen Stelle am Dialysegerät angebracht werden, in vorteilhafter Weise aber so, dass er gut einlesbar ist.

Statt eines Aufklebers kann eine beliebige andere Form einer dauerhaften Markierung, die eine grafische Kodierung, vorzugsweise als QR-Code ausgeführt, aufweist, erzeugt werden und an das Dialysegerät angebracht bzw. damit versehen werden. Somit wird jedes Dialysegerät individuell durch eine eigene ihr zugeordnete grafische Kodierung markiert. Beispiele für solche nichtflüchtigen Markierung können umfassen: Mit der grafischen Kodierung bedrucktes Papier, welches an dem zu markierenden Teil appliziert ist, sowie Gravierung oder Bedruckung des zu markierenden Teils oder damit verbundener Teile, beispielsweise in Form eines Anhängers (Tag), die als grafische Kodierung ausgeführt ist.

Wesentlich ist nicht die Ausführungsform der dauerhaften Markierung, sondern deren einfache Herstellung, deren Unveränderlichkeit, sobald sie hergestellt wurde, und die unverwechselbare Zuordnung zu dem markierenden Dialysegerät. Eine solche dauerhafte Markierung unterscheidet sich somit grundsätzlich von einer auf einer Anzeigevorrichtung angezeigten grafischen Kodierung, die beliebig veränderlich ist und beispielsweise bei ausgeschalteter Anzeigevorrichtung nicht angezeigt werden kann.

Die Information, die in der grafische Kodierung enthalten ist, kann verschlüsselt angezeigt sein. Dies kann durch die Verwendung einer sicheren grafischen Kodierung geschehen, beispielsweise mit dem Secure-QR-Code. Der Secure-QR-Code (SQRC) ist ein QR-Code, bei dem Dateninhalte oder Teile davon nicht durch Dritte eingesehen werden sollen. Zu diesem Zweck werden beim SQRC alle kodierten Daten oder ein Teil der enthaltenen Daten verschlüsselt. Die öffentlichen (unverschlüsselten) Daten in einem SQRC können auch mit normalen Lesegeräten wie mobile Computer gelesen werden, während die verschlüsselten Informationen mit diesen normalen Lesegeräten verborgen bleiben. Um diese Information lesen zu können muss das Lesegerät über eine spezielle Software verfügen, die über einen individuellen Schlüssel, beispielsweise ein Kennwort, verfügt, der auch bei der Generierung der verschlüsselten Information zur Verwendung gekommen ist. Dieser Schlüssel kann vom Informationsanbieter individuell an verschiedenen Personengruppen ausgegeben werden.

Innerhalb einer grafischen Kodierung kann durch die Verwendung mehrerer Schlüssel so Informationen verschlüsselt werden, die sich gleichzeitig an eine Vielzahl von Personengruppen richtet. Jede Personengruppe verfügt über einen eigenen Schlüssel und kann demnach nur den Teil der verschlüsselten Informationen entschlüsseln, der für sie vorgesehen ist. Einzelne Personen können darüber hinaus auch über mehrere Schlüssel verfügen.

Im Einklang mit der Lehre der vorliegenden Erfindung können die beschriebenen nichtflüchtigen Markierungen auch für zu der Behandlungsstation gehörende Teile hergestellt und am jeweiligen Teil angebracht oder damit versehen werden.

Eine Behandlungsstation definiert sich in einer Ausführungsform durch das Dialysegerät mit allen für die anstehende Behandlung notwendigen Zubehörteilen, wie Filter, Schlauchsets, medizinische Lösungsbehälter usw., sowie einer Patientenlagerungsvorrichtung, die beispielsweise als Sessel, Liege oder Krankenbett ausgeführt sein kann, und allen für die jeweilige Dialysebehandlung vorgehaltenen Zusatzgeräte, wie beispielsweise EKG-Geräten, Blutdruckmonitore oder Ähnliches.

Die jeweilige grafische Kodierung ist hierbei typischerweise individuell und charakteristisch für das jeweilig damit versehene Teil. Darüber hinaus kann abgespeichert werden, wenn Teile, die eine grafische Kodierung tragen, zusammenwirken. Dies kann im Sinne der vorliegenden Offenbarung dadurch geschehen, in dem durch einen externen Computer alle Teile einer Behandlungsstation durch Einlesen der grafischen Kodierung identifiziert werden und die so gewonnenen Datensätze miteinander verknüpft werden. Die Information über verknüpfte Teile einer Behandlungsstation kann per Datenfernübertragung an ein entferntes Gerät, beispielsweise an einen Servercomputer gesendet werden.

Auf diese Weise wird an einer zentralen Stelle, nämlich dem Servercomputer, die Information generiert, welche Teile einer Behandlungsstation zusammenwirken. Auch diese Information kann an einen mobilen Computer gesendet werden, wenn dieser zuvor mindestens ein Teil der Behandlungsstation durch Einlesen der applizierten grafischen Kodierung identifiziert hat und das Identifikationsmerkmal wie weiter oben beschrieben an den Servercomputer gesendet hat, da alle Teile der Behandlungsstation datentechnisch im Servercomputer miteinander verknüpft sind.

Eine typische Vorgehensweise zur eindeutigen Identifikation des Patienten während einer Dialysebehandlung sieht vor, dass sich der Patient, beispielsweise durch das Einlesen seiner Patientenkarte, am Dialysegerät bekannt macht. Das Dialysegerät sendet anschließend per Datenfernübertragung die Identifikations-merkmale des Patienten und des Dialysegeräts an einen Servercomputer. Im Servercomputer, der als Zuordnungseinheit konfiguriert ist, werden die Identität des Patienten und die des Dialysegeräts gepaart, also miteinander verknüpft. Zusätzlich können dieser Identitätspaarung auch weitere Datensätze zugeordnet werden. Solche Datensätze können Informationen umfassen, die patienten-individuelle therapeutische bzw. medizinische und/oder physiologische Daten betreffen, oder Daten, die das Dialysegerät, dessen Ausrüstung oder die Dialysestation, in der das Dialysegerät steht, betreffen.

Das medizinisches Personal, welches einen externen Computer, beispielsweise als SmartPhone oder TabletPC mit integrierter Kamera ausgeführt, mit sich führt, kann mit der Kamera einen grafischen Kode, der an einem Teil der Behandlungsstation appliziert ist, einlesen. Durch eine entsprechende Programmierung dekodiert der externe Computer den grafischen Kode und erhält somit ein Identifikationsmerkmal dieses Teils. Dieses Identifikationsmerkmal kann anschließend per Datenfernübertragung an den Servercomputer übertragen werden. Der Datenaustausch zwischen mobilem Computer oder Dialysegerät und Servercomputer kann drahtlos erfolgen. Geeignete drahtlose Datenübertragungswege sind hierbei eine Funkübertragung wie WLAN und Bluetooth, oder auch Infrarotdatenübertragung. Bei allen möglichen Ausführungsbeispielen werden für die Übertragung von Daten solche Datenübertragungsprotokolle bevorzugt, die sicher bezüglich der Vertraulichkeit, der Authentizität und Integrität sind, wie beispielsweise die Datenübertragungsprotokolle IPSec (Internet Protocol Security) oder VPN (Virtual Private Network) oder OpenVPN.

Weiterhin kann zur Authentifizierung des Dialysegeräts und des mobilen Computers ein Datenübertragungsprotokoll mit sicherer Identifikation der sich austauschenden Geräte, wie beispielsweise Bluetooth oder IrDA (Infrared Data Association) verwendet werden.

In einer bevorzugten Ausführungsform wird die grafische Kodierung, die am Dialysegerät, oder die an der Patientenlagerungsvorrichtung appliziert ist, eingelesen, da beide Vorrichtungen bei den üblichen Behandlungsformen in der Regel immer der gleichen Behandlungsstation zugeordnet sind.

Nachdem der Servercomputer vom mobilen Computer das eingelesene Identifikationsmerkmal erhalten hat, kann dieser die Autorisierung des mobilen Computers überprüfen. Dieses kann beispielsweise durch das Überprüfen eines mitgesendeten Identifikationsmerkmals des mobilen Computers, wie eine feste IP-Adresse oder eine MAC-Adresse, geschehen. Im Servercomputer sind zu diesem Zweck die Identifikationsmerkmale mehrerer mobiler Computer zusammen mit damit verknüpften Autorisierungsinformationen hinterlegt.

Auf diese Weise kann festgestellt werden, ob der mobile Computer zum Empfang von Datenpaketen und/oder zum Empfang welcher Datenpakete der mobile Computer autorisiert ist.

In Abhängigkeit der Autorisierung kann der Servercomputer Datenpakete an den mobilen Computer zurücksenden. Diese Daten können Informationen umfassen, die patientenindividuelle therapeutische bzw. medizinische und/oder physiologische Daten betreffen, oder Daten, die das Dialysegerät, dessen Ausrüstung oder die Dialysestation, in der das Dialysegerät steht, betreffen. Nachfolgend kann der Benutzer des mobilen Computers Zugriff auf diese Daten haben.

Beispielsweise kann der Name des Patienten auf dem Display des mobilen Computers angezeigt werden, oder die Belegung der Dialysestation, oder ein Überblick über die vergangenen Dialysebehandlungen des Patienten in Form einer Behandlungshistorie.

Auf diese Weise ist es alleine durch das Einlesen einer grafischen Kodierung an der Behandlungsstation möglich, dem medizinischen oder technischen Personal umfangreiche Informationen zur Verfügung zu stellen, ohne das eine direkte Kommunikation zwischen dem Dialysegerät und dem externen mobilen Computer notwendig ist.

Dies hat insbesondere Vorteile hinsichtlich der Sicherheit der Dialysebehandlung. Ein Datenaustausch zwischen dem Dialysegerät und einem externen mobilen Computer würde eine Gefahr der ungewollten Einflussnahme auf die Dialysebehandlung bergen beispielsweise durch Schadprogramme, die auf dem mobilen Computer unbeabsichtigt laufen.

Derartige Schadprogramme sind beispielsweise Virenprogramme oder Trojanerprogramme, die, vom Benutzer unbemerkt, im mobilen Computer ausgeführt werden können und die häufig das Ziel haben, dem Computer oder der Programmierung des Computers Schaden zuzuführen, oder persönliche Daten, wie Passwörter, auszuspionieren und an Dritte zu übermitteln.

Derartige Programme können sich bei der Datenfernübertragung auf andere Geräte übertragen und dort Schaden anrichten. Gemäß einer Ausführungsform im Einklang mit der Lehre der vorliegenden Erfindung keine die unmittelbare Übertragung derartiger Schadprogramme vom mobilen Computer auf das Dialysegerät möglich, da kein unmittelbarer Datenaustausch zwischen ihnen stattfindet. Servercomputer hingegen, die mit den mobilen Computern und den Dialysegeräten kommunizieren unterliegen besondere Sicherheitssorgfalt durch speziell dafür ausgebildetes Fachpersonal.

So sind diese in der Regel immer mit aktuellen Programmen zur Abwehr von Schadprogrammen ausgestattet und deshalb besonders sicher. Es kann somit mit hoher Wahrscheinlichkeit ausgeschlossen werden, dass sich Schadprogramm über den Servercomputer weiter ausbreiten können.

Das vorgeschlagene Verfahren zur Identifikation eines Patienten und/oder zur einfachen Gewinnung von Informationen über einen Patienten, oder über eine an ihm durchgeführte Behandlung, ist auf jede Behandlungsstation übertragbar, unabhängig davon, ob diese mit einem Fluidmanagementgerät im Zusammenhang steht. So kann eine Behandlungsstation auch ein Krankenbett innerhalb eines Krankenhauses sein, an das eine Grafikkodierung appliziert wird, die ein eindeutiges Identifikationsmerkmal des Krankenbetts kodiert. Ein Arzt oder sonstiges medizinisches Personal kann in schon beschriebener Weise Informationen bezüglich der Identität des Patienten, der in diesem Krankenbett liegt, oder weitere Informationen die Behandlung des Patienten betreffend erhalten. Hierzu wird wie schon beschrieben in einem Servercomputer die Zuordnung zwischen Patient und Krankenbett und gegebenenfalls weitere Informationen, die dem Patienten und/oder dem Krankenbett zugeordnet sind, abgespeichert.

Nachdem die Grafikkodierung mit einem mobilen Computer eingelesen und dekodiert wurde, kann das eindeutige Identifikationsmerkmal des Krankenbetts vom mobilen Computer an den Servercomputer gesendet werden, der in der Folge die dem Patienten bzw. dem konkreten Krankenbett zugeordnete Informationen an den mobilen Computer zurücksenden kann.

Die Herstellung der Zuordnung des Patienten zum Krankenbett bzw. zur Behandlungsstation im Servercomputer, der als Zuordnungseinheit konfiguriert ist, erfolgt beispielsweise während einer zentralen Erfassung der Patientendaten bei der Aufnahme des Patienten im Krankenhaus.

Eine weitere Ausführungsform sieht vor, dass eine grafische Kodierung im Dialysegerät dynamisch erzeugt wird und auf der Anzeigevorrichtung, die oftmals als Touchscreendisplay ausgeführt ist, angezeigt wird.

Die Informationen, die hierdurch kodiert werden, können beliebige Informationen sein. Besonders bevorzugt werden aber Informationen kodiert, die für ein medizinisches Fluidmanagementgerät oder eine damit ausgeführte Behandlung spezifisch sind.

Die Informationen, die dynamisch erzeugt werden, können in der schon beschriebenen Weise verschlüsselt kodiert werden. Auf diese Weise kann sichergestellt werden, dass die Informationen nur von dafür berechtigten Personen gelesen werden können.

Generell kann es vorkommen, dass die Informationsfülle, die innerhalb einer einzigen Grafikkodierung kodiert werden kann, nicht ausreicht, um alle erwünschten Informationen zu kodieren.

Es ist daher eine weitere Aufgabe der Erfindung, ein Verfahren zu schaffen, mit dem sich die Informationsfülle, die grafisch kodiert wird, weiter erhöhen lässt.

Die Lösung dieser Aufgabe erfolgt erfindungsgemäß durch ein Verfahren mit den Merkmalen des Anspruchs 20. Eine vorteilhafte Ausführungsform ist Gegenstand des Unteranspruchs 21. Demnach ist vorgesehen, dass unterschiedlich Grafikkodierungen über eine Anzeigeperiode nacheinander angezeigt werden. Auf diese Weise können mehrere einzelne unterschiedliche Grafikkodierungen hintereinander angezeigt werden. Jede einzelne Grafikkodierung kann unterschiedliche Daten kodieren. Auf diese Weise kann die Fülle der Information, die angezeigt werden soll, erhöht werden. Eine Anzeigeperiode umfasst die Dauer der Anzeige von verschiedenen Grafikkodierungen ohne Wiederholung, wobei die verschiedenen Grafikkodierungen typischerweise zeitnah, oder ohne beabsichtigte Pause nacheinander angezeigt werden Eine solche Abfolge von einzelnen Grafikkodierungen entspricht einem Film, analog der Anzeige von einzelnen Bildern. Die derart angezeigten Grafikkodierungen kodieren jeweils Teile einer zusammengehörenden Information.

In einer weiteren Ausführungsform kann die Grafikkodierung unkodierte grafische Symbole enthalten. Es ist zur Kodierung von Daten mit einer Grafikkodierung nicht immer notwendig, die gesamte zur Verfügung stehende Anzeigefläche zur Kodierung dieser Daten zu verwenden. Es ist demnach möglich, einen Teil der Anzeigefläche für andere Zwecke zu benutzen, beispielsweise zur Anzeige von grafischen Symbolen oder von Klartext. Grafische Symbole können insbesondere Firmenlogos, Bilder oder Piktogramme umfassen.

Werden in schon beschriebener Weise mehrere Grafikkodierungen im Sinne eines Filmes hintereinander angezeigt, kann jede einzelne Grafikkodierung mit einem anderen unkodierten Bild versehen werden. Auf diese Weise kann ein für den Betrachter sichtbarer bewegter Film angezeigt werden.

Ein derartiger Film kann beispielsweise Symbole umfassen, die dem Betrachter die Dauer des Filmes, also den Beginn und das Ende symbolisieren. Ein solches Symbol kann beispielsweise eine Sanduhr mit veränderlichem Sandstand sein.

Auf diese Weise kann dem Betrachter symbolisiert werden, wie lange er die Kamera bzw. die Einlesevorrichtung des mobilen Computers auf die angezeigten Grafikkodierungen halten muss, um alle kodierten Informationen einzulesen. Der angezeigte Film aus Symbolen kann aber auch zu Werbezwecken benutzt werden.

Die Initiierung der Anzeige der Grafikkodierung kann auch durch ein vom mobilen Computer ausgesendetes Signal erfolgen. Ein solches Signal ist in einer Ausführungsform ein optisches Signal, beispielsweise ein Blitzlicht, welches oftmals Bestandteil von Smartphones oder Tablet-PCs ist. Das Dialysegerät kann mit einem entsprechenden Sensor (Photodetektor, Kamera) die Aussendung dieses Signals erkennen und nachfolgend die Anzeige einer Grafikkodierung veranlassen.

In einer weiteren Ausführungsform kann die Anzeige von Grafikkodierungen am Dialysegerät auch durch die Anzeige von Grafikkodierungen am mobilen Computer, die vom Dialysegerät eingelesen wird, initiiert werden. Hierzu wird ein auf der Anzeigevorrichtung des mobilen Computers angezeigte oder als Aufkleber am mobilen Computer applizierte Grafikkodierung einer entsprechenden Lesevorrichtung am Dialysegerät, die eine Kamera umfassen kann, vorgehalten.

Das Dialysegerät verfügt in diesem Fall über eine entsprechende Dekodierungs- und gegebenenfalls über eine Entschlüsselungssoftware, die die eingelesene Grafikkodierung dekodiert bzw. entschlüsselt. Dies hat den Vorteil, dass in der eingelesenen Grafikkodierung auch die Identität des Benutzers des mobilen Computers kodiert sein kann, und die Generierung und die Anzeige von Grafikkodierung am Dialysegerät demnach benutzerindividuell gestaltet werden kann.

Aber auch in jeder weiteren schon beschriebenen Weise kann sich der Benutzer am Dialysegerät bekannt machen. Abhängig von der Identität des Benutzers kann das Dialysegerät verschiedene personenindividuelle oder personengruppen-individuelle Grafikkodierungen erzeugen, die wiederum zusätzlich verschlüsselt sein können.

Dadurch, dass zwischen Dialysegerät und mobilem Computer in manchen Ausführungsformen keine unmittelbare Netzwerkverbindung erfolgt, kann auch keine unmittelbare Beeinflussung des Dialysegeräts durch den mobilen Computer erfolgen. Insbesondere können Schadprogramme nicht unmittelbar übertragen werden. Dies erhöht die Sicherheit der Behandlung.

Im Einklang mit der Lehre der vorliegenden Erfindung kann die dynamisch erzeugte Grafikkodierung alle Informationen kodieren, die in schon beschriebener Weise mit einer als Aufkleber ausgeführten (statischen) Grafikkodierung kodiert werden können.

Die Information, die in der angezeigten Grafikkodierung kodiert ist, kann beispielsweise umfassen:
Hyperlinks, die auf Internetadressen verweisen. Solche Internetadressen können Seiten adressieren, auf denen Datenbanken enthalten sind, die für das jeweilige Gerät spezifisch sind. Sie können aber auch auf Expertenforen oder Expertendatenbanken (ExpertenWiki) verweisen, oder auch auf Seiten, die Verbrauchsmaterial oder Ersatzteile oder Servicedienstleistungen anbieten.

Behandlungbezogene Daten, wie physiologische Werte oder Geräteeinstellungen. Durch die kodierte Darstellung von derartigen Daten kann die unkodierte Anzeige anderer Daten auf der Anzeigevorrichtung reduziert werden. Es ergibt sich somit ein für den Patienten aufgeräumteres, weniger verwirrendes Bild auf der Anzeigevorrichtung. Manchmal liegen auch sogenannte Voralarme bei der Behandlung vor, die wenn sie als Klartext angezeigt werden, den Patienten beunruhigen könnten. Durch die Kodierung im Grafikkode wird diese Beunruhigung des Patienten vermieden.

Darüber hinaus können persönliche Daten des Patienten für den menschlichen Betrachter ohne Hilfsmittel unlesbar gemacht werden. Das medizinische Personal hingegen verfügt mit einem entsprechend programmierten mobilen Computer über ein Mittel, die kodierten Daten lesbar zu machen. Somit wird die Privatsphäre des Patienten geschützt und gleichzeitig die Anzeige von medizinischen Daten für dafür legitimierte Personen ermöglicht.

In einer Ausführungsform kann der Detaillierungsgrad der kodierten Daten entsprechend der Legitimation des Benutzers unterschiedlich sein. So kann beispielsweise die Anzeige für einen Dialysearzt detaillierter sein, als für eine Dialyseschwester. Bei der Generierung der dynamischen Grafikkodierung nachdem sich der Benutzer in beliebiger Weise dem Dialysegerät bekannt gemacht hat, können auch die Art der Daten, die in der Grafikkodierung kodiert sind, unterschiedlich sein. Meldet sich ein Servicetechniker an, werden bevorzugt technische Daten kodiert, meldet sich medizinisches Personal an, werden bevorzugt medizinische Daten kodiert.

Manche Behandlungen werden auch nachts durchgeführt, oder während einer Phase, in der der Patient schläft. Hierbei ist es sinnvoll, die Helligkeit der Anzeigevorrichtung zu reduzieren. Durch die Anzeige einer Grafikkodierung, die alle im normalen Betrieb im Klartext angezeigten Informationen enthält, kann die restliche Anzeigefläche komplett dunkel geschaltet werden. Somit wird der Patient nur minimal gestört. Die Grafikkodierung kann hierfür zusätzlich im Kontrast reduziert sein.

In einer weiteren Ausführungsform kann die Grafikkodierung auch auf einer weiteren gesonderten Anzeigevorrichtung am Dialysegerät angezeigt werden. Mithilfe einer speziell dafür ausgebildeten Halterung kann ein mobiler Computer permanent mit seiner Lesevorrichtung auf die Grafikkodierung ausgerichtet werden. So können Daten vom Dialysegerät zum externen Computer übertragen werden, die dort dekodiert und weiterverarbeitet werden können, ohne dass eine Rückwirkung vom mobilen Computer auf das Dialysegerät erfolgen kann. Die Weiterverarbeitung der Daten im mobilen Computer kann die Anzeige der Information einer Anzeigevorrichtung des mobilen Computers umfassen, sie kann aber auch die Weiterleitung von dekodierter Information an weitere Geräte, beispielsweise an einen Servercomputer, einen Stationsrechner oder an einen Pager von medizinischem Personal umfassen. Dies ist insbesondere bei Alarmmeldungen von Vorteil.

Die angezeigte Grafikkodierung kann auch Informationen über benötigtes Verbrauchsmaterial, anstehende Servicetätigkeiten oder notwendige Reparaturen enthalten. Der mobile Computer kann so programmiert sein, dass er nach erfolgter Dekodierung und gegebenenfalls Entschlüsselung der Information von sich aus Bestellungen abgibt oder Servicetechniker bestellt. Dies kann beispielsweise per SMS (Small Message Service) oder Email-Nachricht an einen Servicetechniker oder durch eine automatisierte Bestellung bei eine bestimmte Anbieter von Verbrauchsmaterial erfolgen. Der mobile Computer kann hierbei auf das Internet zugreifen und/oder über eine Mobilfunkeinrichtung verfügen. Dies ist insbesondere dann von Vorteil, wenn der die Behandlung beim Patienten zuhause geschieht, wie oftmals bei der Peritonealdialyse. In manchen Fällen erfolgt auch eine Hämodialysebehandlung beim Patienten zuhause. Der Benutzer des mobilen Computers ist in solchen Fällen oftmals der Patient selbst, dem die Kompetenz, auf sämtliche Meldungen des Dialysegeräts richtig zu reagieren, in der Regel fehlt.

Hierbei können bei der Heimdialyse auch behandlungsbezogenen Daten an Dritte, beispielsweise medizinisches Personal übertragen werden, die im Bedarfsfall entsprechend reagieren können. Beispielsweise kann ein Dialysearzt nach Auswertung der übermittelten Daten in Kontakt mit den Patienten treten, um weitere Maßnahmen mit ihm zu besprechen.

In einer weiteren Ausführungsform kann der mobile Computer mit einer Lesevorrichtung, beispielsweise einer Kamera, die als Aufkleber an Teilen der Behandlungsstation applizierten Grafikkodierungen einlesen und dekodieren. Auf diese Weise kann die Information gewonnen werden, welche Teile zu einer Behandlungsstation gehören. Diese Information kann in der jeweils applizierten Grafikkodierung enthalten sein. Der mobile Computer kann seinerseits eine Grafikkodierung generieren, die die Information kodiert, welche Teile eingelesen worden sind. Diese Grafikkodierung kann ihrerseits vom Dialysegerät durch Vorhalten, Einlesen und Dekodieren der angezeigten Grafikkodierung vor eine Lesevorrichtung, beispielsweise eine Kamera, zum Dialysegerät übertragen werden.

Zur Generierung von gegebenenfalls verschlüsselten Grafikkodierungen und zur Dekodierung und gegebenenfalls Entschlüsselung von Grafikkodierungen sind sowohl das Dialysegerät also auch der mobile Computer spezifisch programmiert.

Diese spezifischen Programmierungen sind Computerprogramme und können direkt in einen internen Speicher des mobilen Computers und/oder des Dialysegeräts geladen werden und Softwarecodeabschnitte umfassen, die die beschriebenen Verfahren ausführen, wenn die Programme auf dem mobilen Computer oder dem Dialysegerät laufen. Die Computerprogramme können als Computerprogrammprodukte, die computerlesbare Programmmittel umfassen, auf Datenmedien vorgehalten werden. Diese Datenmedien sind in einem Computer einsetzbar und umfassen neben physikalischen Speichern, wie Disketten, CD-Roms, Speicherkarten, USB Sticks oder DVDs auch die Speicherung innerhalb von Netzwerken, wie dem Internet, auf die der Benutzer Zugriff haben kann.

### Kurze Beschreibung der Figuren

Weitere Einzelheiten und Vorteile der Erfindung werden anhand von in den Zeichnungen dargestellten Ausführungsbeispielen näher beschrieben. Es zeigen:
Die Figur 1 ein medizinisches Fluidmanagementgerät im Einklang mit der Lehre der vorliegenden Erfindung, beispielhaft als Dialysegerät ausgeführt;
die Figur 2 eine Ausführungsform für den Bildschirminhalt eines medizinischen Fluidmanagementgeräts im Einklang mit der Lehre der vorliegenden Erfindung;
die Figur 3 eine weitere Ausführungsform für den Bildschirminhalt eines medizinischen Fluidmanagementgeräts im Einklang mit der Lehre der vorliegenden Erfindung;
die Figur 4 eine Ausführungsform eines mobilen Computers, hier als Smartphone ausgeführt, mit beispielhaftem Bildschirminhalt im Einklang mit der Lehre der vorliegenden Erfindung;
die Figur 5 eine symbolhafte Darstellung eines Ablaufs der Anzeige von mehreren unterschiedlichen Grafikkodierungen mit einem enthaltenen veränderlichen Symbol im Einklang mit der Lehre der vorliegenden Erfindung;
die Figur 6 eine symbolhafte Darstellung der angezeigten Grafikkodierung und der daraus dekodierten Information, die die Ausrüstung und den Zustand eines Dialysegeräts betrifft, im Einklang mit der Lehre der vorliegenden Erfindung;
die Figur 7 eine symbolhafte Darstellung einer Behandlungsstation mit applizierten Aufklebern, die eine Grafikkodierung aufweisen, im Einklang mit der Lehre der vorliegenden Erfindung;
die Figur 8 ein Ablaufdiagramm eines Verfahrens zur Bestimmung zumindest der Patientenidentität, im Einklang mit der Lehre der vorliegenden Erfindung;
die Figur 9 eine symbolhafte Darstellung eines Systems aus einem medizinischen Gerät, einem mobilen Computer und einem weiteren Computer zur Bestimmung zumindest der Patientenidentität, im Einklang mit der Lehre der vorliegenden Erfindung;
die Figur 10 ein Ablaufdiagramm eines Verfahrens zur Übermittlung und Anzeige von Informationen, die für ein medizinisches Fluidmanagementgerät oder einer damit ausgeführten Behandlung spezifisch sind, auf einem mobilen Computer, im Einklang mit der Lehre der vorliegenden Erfindung.

### Ausführliche Beschreibung der Figuren

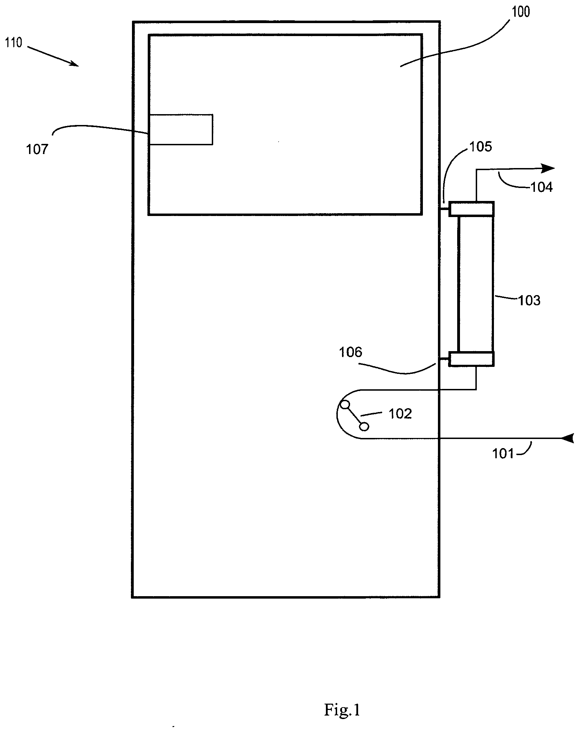

In Figur 1 ist eine Ausführungsform eines medizinischen Fluidmanagementgerätes 110 als Hämodialysegerät mit einem Touchscreendisplay 100 schematisch dargestellt. Das Dialysegerät 110 zeigt andeutungsweise Teile eines extrakorporalen Blutkreislaufs mit einer arteriellen Blutleitung 101, die Blut eines Patienten (nicht dargestellt) ableitet. Die Blutpumpe 102 fördert das Blut durch einen Dialysefilter 103, der mit einer semipermeablen Membran ausgestattet ist, die den extrakorporalen Blutkreislauf von einem Dialysatkreislauf semipermeabel trennt. Über die venöse Leitung 104 wird das behandelte Blut dem Patienten zurückgegeben. Über die Dialysatleitungen 105 und 106 wird Dialysat durch den Dialysefilter 103 gepumpt, wo es über die semipermeable Membran des Dialysefilters 103 zu einem diffusiven Stoffaustausch mit dem Blut des Patienten kommt. Wird zusätzlich ein Druckgradient von der Blutseite des Dialysefilters zur Dialysatseite des Patienten aufgebaut, wird Plasmawasser aus dem Blut in das Dialysat abgepresst. Das abgepresste Plasmawasser wird auch Ultrafiltrat genannt. Das Blut des Patienten kann so entwässert werden. Das Dialysat wird in dem Hämodialysegerät 110 hergestellt und nach Gebrauch verworfen. Das Touchscreendisplay 100 weist eine Teilfläche 107 auf, auf der im Einklang mit der Lehre der vorliegenden Erfindung grafische Codes angezeigt werden können.

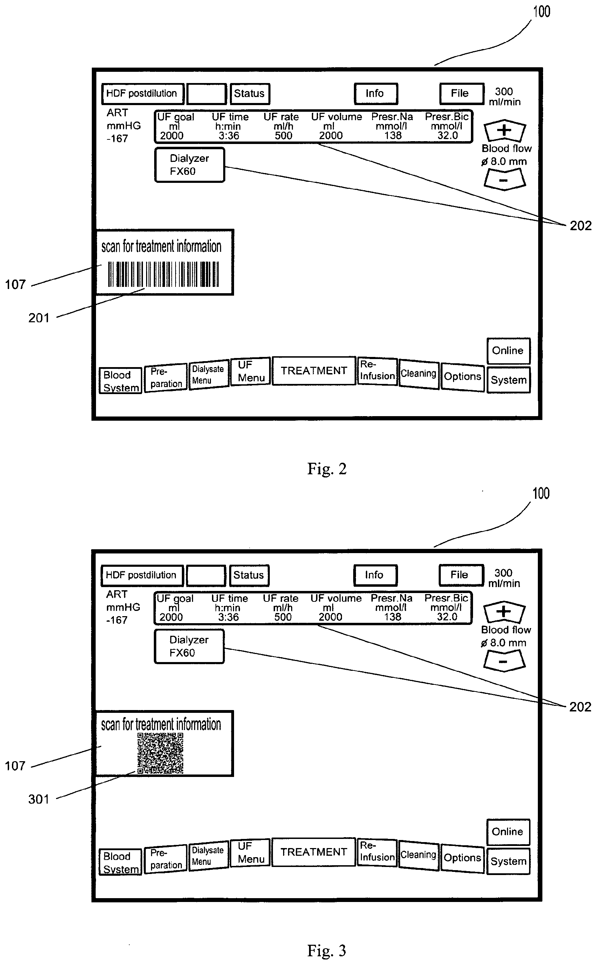

In der Figur 2 ist das Touchscreendisplay 100 mit einem beispielhaften Bildschirminhalt Einklang mit der Lehre der vorliegenden Erfindung näher dargestellt.

Dieser beispielhafte Bildschirminhalt ist typisch für ein Hämodialysegerät und zeigt die Situation am Ende der Behandlung. Das Bezugszeichen 202 weist auf Informationsanzeigen, die für eine Hämodialysebehandlung typisch sind und diese charakterisieren. Diese Informationsanzeigen geben beispielsweise Auskunft über die Gesamtmengen an entzogenem Ultrafiltrat, den verwendeten Dialysefilter oder die Behandlungsdauer.

In den Figuren 2 und 3 erkennt man das Behandlungsende daran, dass die Werte der Anzeigen für das Ultrafiltrationsziel (UF goal) und die aktuelle Ultrafiltratmenge (UF volume) übereinstimmen.

Im Feld 107 wird ein grafischer Code, der in Figur 2 als Strichcode 201 und in Figur 3 als QR Code 301 ausgeführt ist, angezeigt. Es sind darüber hinaus sämtliche grafischen Kodierungen denkbar, die angezeigt werden können, beispielsweise auch ein Flickercode. Der Flickercode arbeitet mit Lichtimpulsen, die Daten kodieren können und ist beispielsweise durch das ChipTAN-Verfahren im Online-Bankingwesen bekannt.

Im Einklang mit der Lehre der vorliegenden Erfindung ist vorgesehen, dass die angezeigte Grafikkodierung Informationen kodieren kann, die für das medizinische Fluidmanagementgerät, das in der Figur 1 als Dialysegerät ausgeführt ist, oder einer damit ausgeführten Behandlung spezifisch sind.

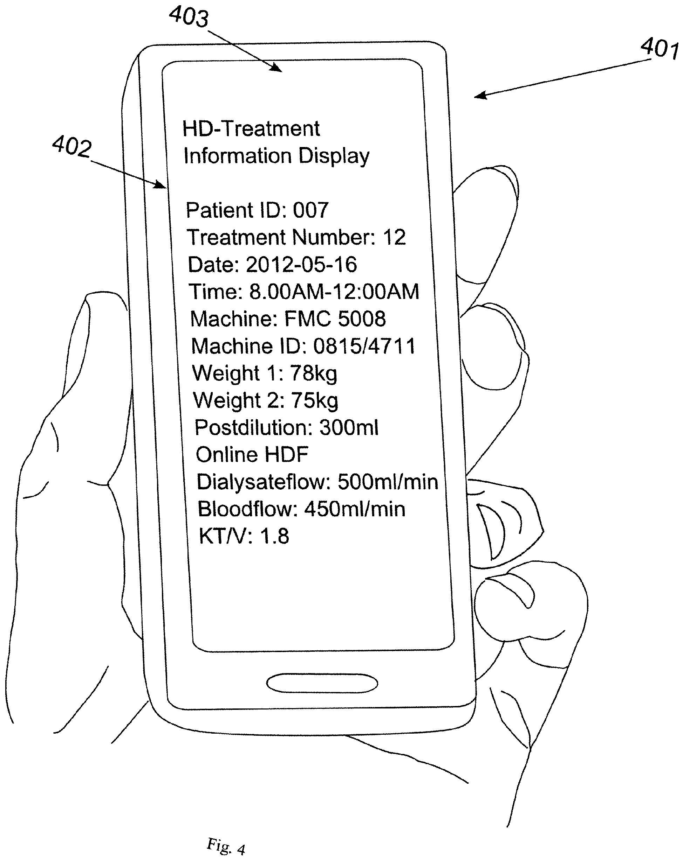

Die Figur 4 zeigt ein als Smartphone ausgeführten mobilen Computer 401, auf dessen Anzeigevorrichtung 403 beispielhafte Informationen angezeigt werden, die auf Grund der Grafikkodierung 201 oder 301 ermittelt wurden. Der Bediener hat zuvor mit einer Einlesevorrichtung des Geräts 401 die Grafikkodierung 201 oder 301 eingelesen. Eine solche Einlesevorrichtung ist typischerweise eine Kamera (in Figur 4 nicht dargestellt). Smartphones sind heutzutage überwiegend mit zumindest einer Kamera ausgerüstet.

Statt eines Smartphones kann das transportable Gerät auch ein Tablet-PC oder ein speziell für diesen Zweck konstruiertes Gerät sein.

Die Steuervorrichtung des mobilen Computers 401 dekodiert unter Verwendung einer dafür vorgehaltenen Software die Informationen, die durch die eingelesenen Grafikkodierungen 201 oder 301 kodiert sind und kann diese auf dem Display anzeigen. Es findet somit eine Weiterverarbeitung der durch die Grafikkodierung kodierten Informationen statt. Diese Weiterverarbeitung der Informationen kann neben der bloßen Anzeige der Information auch die Speicherung der Informationen, das Versenden der Informationen per Datenübertragung, beispielsweise als Email, oder die grafische Darstellung des Verlaufs der die Behandlung betreffenden Informationen sein. Die Möglichkeiten der Weiterverarbeitung der Informationen sind darüber hinaus so vielfältig, wie es die Ausstattung des transportablen Geräts 401 in Verbindung mit der hinterlegten Software ermöglicht.

Die in der Figur 4 beispielhaft angezeigten Informationen betreffen sowohl den Patienten, der durch einen Patientennummer (Patient ID) gekennzeichnet ist, als auch die Umstände der Behandlung, wie Datum (Date), Uhrzeit der Behandlung (Time), Typ und laufende Nummer des Dialysegeräts (Machine, Machine ID). Weiterhin werden bestimmte behandlungsbezogene Informationen angezeigt, wie Gewicht des Patienten am Anfang der Behandlung (Weight 1) und Gewicht am Ende der Behandlung (Weight 2), Art der Behandlung (HDF entsprechend Hämodiafiltration) mit Postdilution (Postdilution), Dialysatflussrate (Dialysateflow), Blutflussrate (Bloodflow) und Dialysedosis (KT/V). Die Dialysedosis ist für die Effektivität einer Dialysebehandlung von entscheidender Bedeutung und setzt sich aus dem mit dem Harnstoffverteilungsvolumen V des Patienten bewerteten Produkt der Clearance K des Dialysefilters und der abgelaufenen Zeit T zusammen. Die DE 10 2006 032 926 der Anmelderin macht hierzu weitere Angaben.

Vorteilhaft im Einklang mit der Lehre der vorliegenden Erfindung ist, dass diese Informationen, die von der Anzeigevorrichtung 403 angezeigt werden, nicht unmittelbar in der Grafikkodierung 201 oder 301 kodiert sein müssen. Es reicht aus, dass ein für das medizinische Fluidmanagementgerät charakteristisches Identifikationsmerkmal in der Grafikkodierung 201 oder 301 kodiert ist. Der mobile Computer kann dieses charakteristische Identifikationsmerkmal dekodieren und an einen entfernten Computer, beispielsweise einen Server, übermitteln, in dem die anzuzeigenden Informationen mit dem charakteristischen Identifikationsmerkmal verknüpft sind. Hierzu kann der entfernte Computer die entsprechenden Informationen zuvor per Datenfernübertragung vom medizinischen Fluidmanagementgerät selbst erhalten. Der entfernte Computer sendet diese Informationen an den mobilen Computer, wo sie angezeigt werden können. Es findet auf diese Weise keine oder keine unmittelbare Einflussname des mobilen Computers auf das medizinische Fluidmanagementgerät statt, da eine unmittelbare Datenübertragung, vom mobilen Computer zum medizinischen Fluidmanagementgerät nicht erforderlich ist. Die Datenübertragung erfolgt in diesem Beispiel nur vom medizinischen Fluidmanagementgerät zum mobilen Computer optisch durch die Anzeige der Grafikkodierung, bzw. vom medizinischen Fluidmanagementgerät zu einem entfernten Computer, die im Hinblick auf die Integrität der Datenverbindung besonders sicher ausgebildet sein kann, und vom entfernten Computer zum mobilen Computer, aber nicht oder nicht notwendig vom mobilen Computer zum medizinischen Fluidmanagementgerät.

Die in Figur 4 angezeigten Informationen sind nur beispielhaft für eine mit einem Hämodialysegerät durchgeführte Behandlung. Es können auch beliebige andere für das jeweilige medizinische Fluidmanagementgerät und/oder einer damit ausgeführten Behandlung spezifische Informationen angezeigt werden.

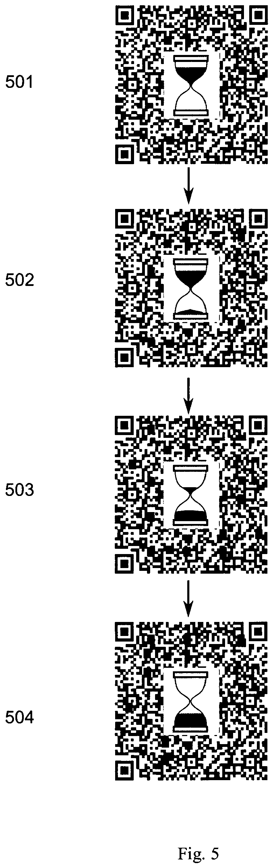

Die Figur 5 zeigt mehrere unterschiedliche QR-Codes 501 bis 504 mit jeweils einem mit dem menschlichen Auge erkennbarem Symbol, hier als Sanduhr mit unterschiedlichem Füllstand ausgeführt.

Die QR-Codes 501 bis 504 werden in einer Sequenz nacheinander angezeigt und kodieren in der Summe mehr Information, als dies mit nur einem einzigen QR-Code möglich wäre. Für den Benutzer ist es hilfreich zu wissen, wann die Sequenz vollständig angezeigt worden ist. Dies wird ihm durch die stilisierte Sanduhr angezeigt, die im Sinne eines Ablaufs den Anfang und das Ende der Sequenz charakterisiert. Es sind beliebige Symbole oder Klartexte möglich, so z.B. auch Firmenlogos zu Werbezwecken, die animiert gestaltet sein können, in dem pro angezeigtem QR-Code ein verändertes Logo angezeigt wird.

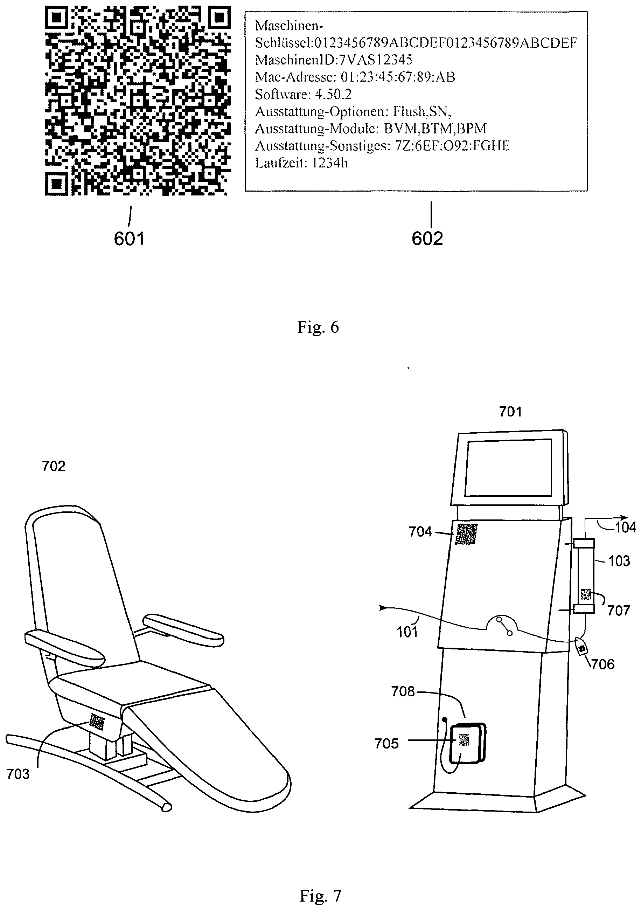

Die Figur 6 zeigt ein Beispiel für einen grafischen Code 601, hier als QR Code ausgeführt, und eine beispielhafte Ausstattungs- und Zustandsliste 602, die durch den Code 601 kodiert ist, als weiteres Ausführungsbeispiel für die Informationen die durch die Grafikkodierung kodiert werden können.

Diese Ausstattungs- und Zustandsliste 602 umfasst die Kennzeichnungen für beispielsweise einen Maschinenschlüssel, die Maschinenidentifikationsnummer, die MAC-Adresse der Steuervorrichtung des medizinischen Geräts, die Softwarenummer, Ausstattungsoptionen, zusätzlich installierte Ausstattungsmodule, sonstige Ausstattungen und die Laufzeit des medizinischen Geräts.

Weiterhin können auch Fehlermeldungen auf diese Weise kodiert und weitergegeben werden. Diese Informationen können in schon beschriebener Weise vom mobilen Computer dekodiert werden und/oder an ein drittes entferntes Gerät, beispielsweise ein Mobiltelefon oder eine E-Mail-Adresse eines Servicetechnikers übermittelt werden und dienen im Servicefall als Information für den Servicetechniker.

Dieser kann sich aufgrund der ihm übermittelten Informationen vorab schon ein Bild vom Fehlerfall bzw. der konkreten Ausgestaltung eines medizinischen Geräts machen und sich dementsprechend auf die Serviceoperation genau vorbereiten und beispielsweise passende Ersatzteile schon zum medizinischen Gerät mitbringen.

In jedem Fall kann die angezeigte Grafikkodierung verschlüsselt ausgeführt sein. Je nach Berechtigung des Benutzers, der diese Grafikkodierung einliest, können unterschiedliche Informationen freigegeben werden.

Die Figur 7 zeigt eine typische Behandlungsstation bestehend aus einer Patientenlagerungsvorrichtung 702, hier als Behandlungssessel ausgeführt, und einem Hämodialysegerät 701 analog der Figur 1. Beispielhaft sind hier verschiedene Teile der Behandlungsstation mit applizierten Grafikkodierungen 703 bis 707 versehen.

Die Grafikkodierungen werden individuell für jedes Teil der Behandlungsstation erzeugt, beispielsweise durch Ausdrucken von QR-Codes auf Aufklebern oder Papier, wobei die relevanten Informationen, wie Geräte- oder Teileidentifikationsnummern, Typ, Baujahr und/oder sonstige Informationen vom QR-Code kodiert werden können, der darüber hinaus verschlüsselt ausgeführt sein kann.

Die Grafikkodierung 703 ist charakteristisch für einen bestimmten Behandlungssessel, 704 für ein bestimmtes Dialysegerät, 705 für ein bestimmtes Zubehörteil 708, hier bespielhaft als Kanister ausgeführt, 706 für einen bestimmten Blutschlauch 101 und 707 für einen bestimmten Dialysefilter 103. Sämtliche Grafikkodierungen können jeweils individuell und für jedes einzelne Teil einzigartig erstellt werden. Auch gleiche Teile wie Dialysefilter können mit einem individuellen Identifikationsmerkmal versehen werden, um eine eindeutige Zuordnung zu ermöglichen.

Im Einklang mit der Lehre der vorliegenden Erfindung kann ein mobiler Computer sämtliche derart gekennzeichnete Teile einer Behandlungsstation einlesen und dekodieren. Anschließen kann der mobile Computer die so gewonnene Information, nämlich welche Teile einer Behandlungsstation zusammen wirken, weiterleiten.

Diese Weiterleitung kann beispielsweise durch eine Datenfernübertragung zu einem entfernten Computer, beispielsweise an einen Server erfolgen. In diesem wird die Information abgelegt. Macht sich bei einer Behandlung der Patient an der Dialysestation bekannt, beispielsweise durch Einlesen seiner Patientenkarte am Dialysegerät, kann das Dialysegerät die Patientenidentität an den entfernten Computer übermitteln, beispielsweise über eine Datenfernübertragung. Zusammen mit der Patientenidentität kann auch ein Identifikationsmerkmal des Dialysegeräts übermittelt werden. Auf diese Weise kann im entfernten Computer die Zuordnung zwischen dem konkreten Dialysegerät und der Patientenidentität erfolgen.

Dadurch, dass gegebenenfalls auch die dem Dialysegerät innerhalb einer Behandlungsstation zugeordneten Teile, die mit einer Grafikkodierung versehen sind, dem Dialysegerät im entfernten Computer bekannt gemacht worden und dort einander zugeordnet sind, genügt es für beispielsweise einen Dialysearzt, eine beliebige Grafikkodierung innerhalb der Behandlungsstation mit einem mobilen Computer einzulesen, um die Identität des Patienten vom entfernten Computer übertragen zu bekommen. Des Weiteren kann neben der Patientenidentität auch eine Fülle weiterer Informationen übertragen werden, beispielsweise Informationen über den aktuellen Behandlungsverlauf, die vom Dialysegerät an den entfernten Computer übertragen worden sind, oder auch die gesamte dem entfernten Computer bekannt gemacht gewordene Ausrüstung der jeweiligen Behandlungsstation. Darüber hinaus kann auch ein Überblick über die Gesamtsituation innerhalb einer Dialysestation mit mehreren Behandlungsstationen übertragen werden, wenn im entfernten Computer diese Daten vorliegen und einander zugeordnet sind.

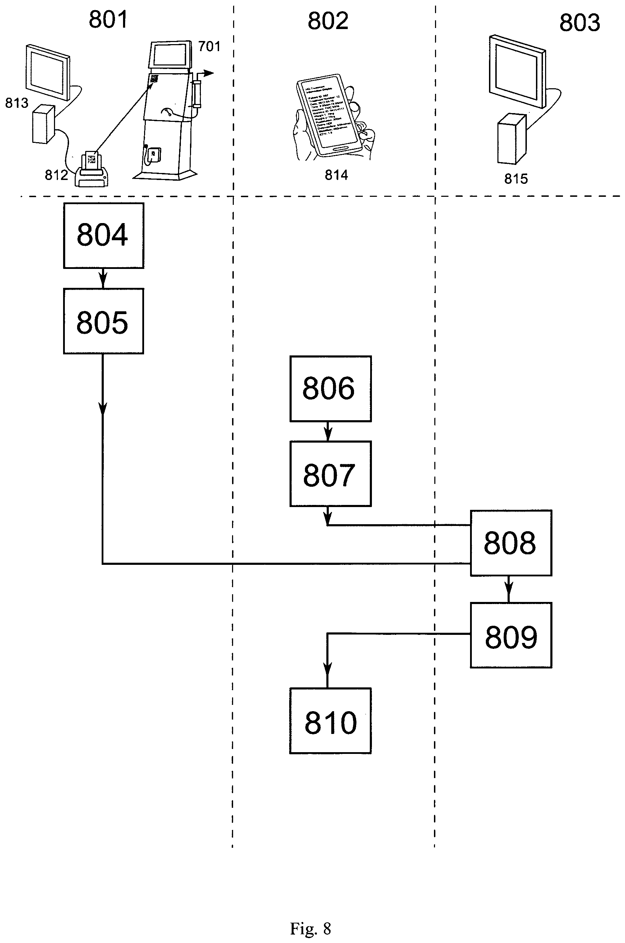

Die Figur 8 zeigt exemplarisch ein Ablaufdiagramm für ein System auf, welches die Anzeige der Patientenidentität auf einem mobilen Computer durch Einlesen einer Grafikkodierung im Einklang mit der Lehre der vorliegenden Erfindung ermöglicht. Die Figur 8 ist in drei Spalten 801, 802 und 803 unterteilt, um zu kennzeichnen, welche Schritte von welchen Geräte ausgeführt werden. An den Schritten, die in Spalte 801 aufgeführt sind, ist ein System aus einem Computer 813, der mit einem Drucker 812 ausgerüstet sein kann, und einem Dialysegerät 701, beteiligt. Die Spalte 802 kennzeichnet Schritte, die von einem mobilen Computer 814, dort beispielhaft als Smartphone ausgeführt, durchgeführt werden. Die Spalte 802 kennzeichnet Schritte, die von einem entfernten Computer 815, der identisch mit dem Computer 812 sein kann, ausgeführt werden.

Zunächst wird im Schritt 804 die für das jeweilige Dialysegerät spezifische Grafikkodierung am Computer 813 erzeugt. Hierzu wird zumindest ein eindeutiges Identifikationsmerkmal, wie individuelle Maschinennummern oder feste IP-Adressen, unter denen Dialysegeräte in einem Datennetz kommunizieren oder MAC-Adressen (Media-Access-Control-Adressen), die die Hardware von Dialyse-geräten eindeutig identifizieren, in ein Computerprogramm, das auf dem Computer 813 ausgeführt wird, eingegeben. Dieses Computerprogramm ist derart programmiert, dass es das Identifikationsmerkmal in eine Grafikkodierung kodiert, beispielsweise einen QR-Code.

Anschließend wird diese Grafikkodierung an dem Dialysegerät appliziert. Dies kann dadurch geschehen, indem ein zuvor mithilfe eines Druckers 813 erstellter Aufkleber, der die Grafikkodierung aufweist, am Dialysegerät angebracht wird.

Im Schritt 805 meldet sich ein Patient an dem Dialysegerät an. Dies kann beispielsweise dadurch geschehen, in dem seine Patientenkarte am Dialysegerät eingelesen wird. Anschließend erfolgt die Übertragung der Patientenidentität zusammen mit dem Identifikationsmerkmal des Dialysegeräts an den entfernten Computer 815.

Im Schritt 806 erfolgt das Einlesen der Grafikkodierung am Dialysegerät 701 mit einer Einlesevorrichtung, beispielsweise einer Kamera, des mobilen Computers. Durch eine entsprechend programmierte Software, die auf dem mobilen Computer läuft, wird die kodierte Information im Schritt 807 dekodiert. Anschließend erfolgt die Übermittlung der Information, also zumindest des Identifikationsmerkmals des Dialysegeräts 701, an den entfernten Computer 815, beispielsweise per Datenfernübertragung.

Im Schritt 808 wird durch eine entsprechend programmierte Software, die auf dem entfernten Computer 815 läuft, geprüft, welche Patientenidentität dem übertragenen Identifikationsmerkmal des Dialysegeräts 701 zugeordnet ist. Im Schritt 809 erfolgt dann die Zusammenstellung eines Datensatz, der zumindest die Patientenidentität umfasst. Zusätzlich können in diesem Datensatz weitere patientenbezogene Informationen enthalten sein, beispielsweise Behandlungsdaten, physiologische Daten o. Ä.

Anschließend werden diese Daten zum externen Computer übertragen und im Schritt 810 dort für den Benutzer aufbereitet und zur Anzeige gebracht.

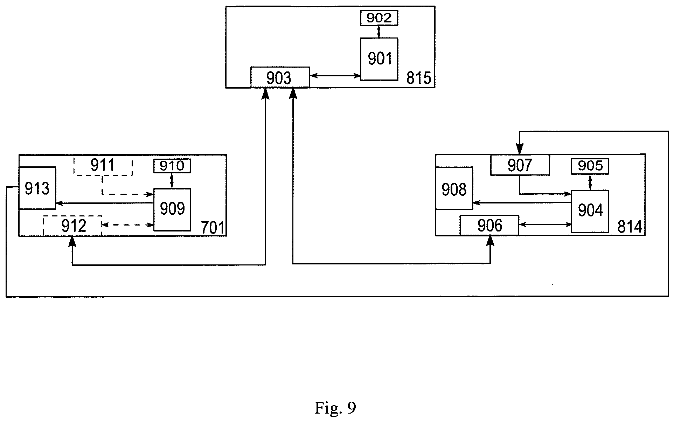

Die Figur 9 zeigt im Einklang mit der Lehre der vorliegenden Erfindung eine Darstellung der Geräte, die die Ausführungsformen der beschriebenen Verfahren ausführen können, und deren Bestandteile.

Hierbei umfassen das Dialysegerät 701, der entfernte Computer 815 und der mobile Computer 814 jeweils zumindest ein Steuergerät 909, 904 und 901, die jeweils auf einen Speicher 910, 902 und 905 zugreifen können.

In diesen Speichern sind jeweils Softwareprogramme abgelegt, die die Steuereinheit derart programmieren, dass sie die beschriebenen Verfahren im Einklang mit der Lehre der vorliegenden Erfindung ausführen können.

Hierzu verfügen der mobile Computer 814 und der entfernte Computer 815 jeweils über eine Datenschnittstelle 906 und 903, über die sie mit anderen Geräten Daten austauschen können. Derartige Schnittstellen können beispielsweise Netzwerkschnittstellen oder Mobilfunkvorrichtungen sein.

Weiterhin verfügt der mobile Computer über mindestens ein Eingabemittel 907, über das Informationen dem Steuergerät 904 zugänglich gemacht werden können. Ein solches Eingabemittel kann eine Kamera sein.

Die an einen Benutzer gerichteten Informationen können über das wenigstens eine Ausgabemittel 908 ausgegeben werden. Ein solches Ausgabemittel kann ein Display sein.

Ein kombiniertes Ein- Ausgabemittel ist beispielsweise ein Touchscreendisplay.

Das Dialysegerät 701 umfasst optional eine Datenschnittstelle 912 und eine Eingabemittel 911, was durch die gestrichelte Darstellung symbolisiert ist. Das wenigstens eine Ausgabemittel 913 kann ein kombiniertes Ein- Ausgabemittel beispielsweise ein Touchscreendisplay sein.

Über die Datenschnittstelle 912 kann das Dialysegerät mit dem entfernten Computer kommunizieren, um ihm beispielsweise eine an der Eingabevorrichtung, die in diesem Fall als Kartenlesegerät konfiguriert ist, eingelesenen Patientenidentität und beispielsweise ein Identifikationsmerkmal zu übermitteln. Dies ist durch den Pfeil zwischen den Datenschnittstellen 912 und 903 symbolisiert.

In gleicher Weise kann der mobile Computer 814 Informationen an den entfernten Computer 815 senden. Beispielsweise kann ein Identifikationsmerkmal, das durch Einlesen, Dekodieren und gegebenenfalls Entschlüsseln einer eingelesenen Grafikkodierung gewonnen wurde, übermittelt werden.

Der entfernte Computer 815 kann ebenso Daten über die Datenschnittstelle 903 an den mobilen Computer versenden, beispielsweise eine mit dem vom mobilen Computer 814 übermittelten Identifikationsmerkmal verknüpfte Patientenidentität.

Die Datenübermittlung vom Dialysegerät 701 zum mobilen Computer 814 geschieht bevorzugt nur über den optischen Weg, beispielsweise von der Ausgabevorrichtung 913 über die Eingabevorrichtung 907 in schon beschriebener Weise, indem eine auf der Ausgabevorrichtung angezeigte Grafikkodierung mit der Eingabevorrichtung 907 eingelesen wird.

Die Grafikkodierung kann im Einklang mit der Lehre der vorliegenden Erfindung auch fest am Dialysegerät appliziert sein, beispielsweise durch einen Aufkleber.

Eine direkte Datenübermittlung vom mobilen Computer 814 zum Dialysegerät 701 ist in einer bevorzugten Ausführungsform im Einklang mit der Lehre der vorliegenden Erfindung nicht vorgesehen, so dass eine Beeinflussung des Dialysegeräts 701 durch den mobilen Computer 814, insbesondere durch Schadprogramme, nicht möglich ist.

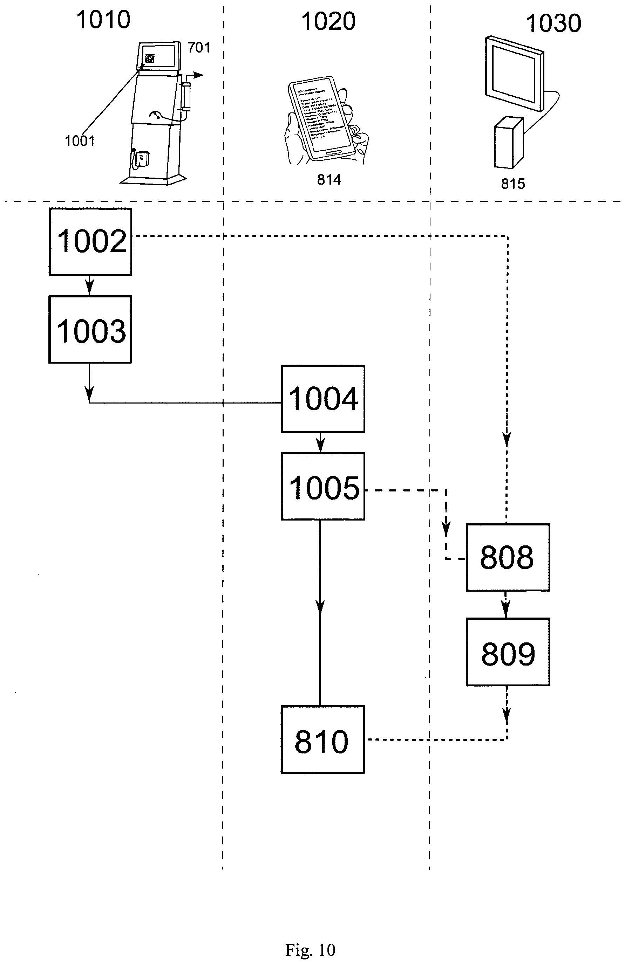

Die Figur 10 zeigt exemplarisch ein Ablaufdiagramm für ein Verfahren, welches die Anzeige von Informationen auf einem mobilen Computer ermöglicht, wobei die Informationen für ein medizinisches Fluidmanagementgerät und/oder einer damit ausgeführten Behandlung spezifisch sind und anhand der Berechtigung der zum Empfang der Information ausgewählten Personengruppe spezifisch verschlüsselt sind, im Einklang mit der Lehre der vorliegenden Erfindung. Die Figur 10 ist in drei Spalten 1010, 1020 und 1030 unterteilt, um zu kennzeichnen, welche Schritte von welchen Geräten ausgeführt werden. An den Schritten, die in Spalte 1010 aufgeführt sind, ist ein Dialysegerät 701 als Beispiel für ein medizinisches Fluidmanagementgerät beteiligt. Die Spalte 1020 kennzeichnet Schritte, die von einem mobilen Computer 814, dort beispielhaft als Smartphone ausgeführt, durchgeführt werden. Die Spalte 1030 kennzeichnet Schritte, die von einem entfernten Computer 815 ausgeführt werden.

Zunächst wird im Schritt 1002 die Grafikkodierung 1001 erzeugt, die für das medizinische Fluidmanagementgerät und/oder einer damit ausgeführten Behandlung spezifisch ist. Diese Grafikkodierung kann zumindest in Teilen in schon beschriebener Weise derart verschlüsselt sein, dass sie nur von solchen Personengruppen entschlüsselt werden können, die hierzu berechtigt sind. Dies wird im Wesentlichen dadurch erreicht, in dem bei der Verschlüsselung ein spezifischer Schlüssel verwendet wird, welcher bei der Entschlüsselung durch einen mobilen Computer, der von der berechtigten Person verwendet wird, zur Verfügung steht.

Im Schritt 1003 wird diese Grafikkodierung 1001 auf einer Anzeigevorrichtung des medizinischen Fluidmanagementgeräts angezeigt.

Im Schritt 1004 erfolgt das Einlesen der Grafikkodierung am Dialysegerät 701 mit einer Einlesevorrichtung, beispielsweise einer Kamera, des mobilen Computers 814. Durch eine entsprechend programmierte Software, die auf dem mobilen Computer läuft, wird die kodierte und verschlüsselte Information im Schritt 1005 dekodiert und gegebenenfalls entschlüsselt. Je nach Berechtigung der einlesenden Person verfügt der mobile Computer hierzu über einen, mehrere oder keinen Softwareschlüssel und kann dementsprechend verschlüsselte Informationen dekodieren und entschlüsseln und/oder öffentliche Informationen dekodieren. In einem nachfolgenden Schritt 810 erfolgt die Anzeige der dekodierten und gegebenenfalls entschlüsselten Informationen.

Die Information, die in der Grafikkodierung 1001 enthalten ist, kann auch die Identität des mit dem medizinischen Fluidmanagementgerät behandelten Patienten enthalten. Eine derartige Information wird bevorzugt verschlüsselt angezeigt. Ein Arzt, der mit einem mobilen Computer 814 die Grafikkodierung 1001 einliest, kann diese Information durch Vorhalten des passenden Schlüssels im mobilen Computer sichtbar machen.

Die Information, die in der Grafikkodierung 1001 enthalten ist, kann auch ein Identifikationsmerkmal des medizinischen Fluidmanagementgerätes enthalten. Analog zu dem in Figur 8 beschriebenen Verfahren kann die Patientenidentität durch Einlesen und Entschlüsseln der Grafikkodierung 1001 und Senden im des Identifikationsmerkmals an einen Computer 815 durch den mobilen Computer 814 und Senden der im Computer 815 dem Identifikationsmerkmal zugeordneten Patientenidentität an den mobilen Computer 814 übermittelt werden. Diese Verfahrensschritte sind optional bzw. alternativ, und sind durch die gestrichelten Linien in Figur 10 gekennzeichnet.

## Patentansprüche

1. Verfahren zur Herstellung einer Zuordnung einer Patientenidentität zu einer Behandlungsstation, wobei die Behandlungsstation ein Dialysegerät (701) und alle für eine anstehende Behandlung notwendige Zubehörteile umfasst, umfassend die Schritte:
Generierung (804) zumindest einer Grafikkodierung, die jeweils ein Identifikationsmerkmal der Behandlungsstation und/oder eines Teils, das mit der Behandlungsstation zusammenwirkt, kodiert,
Applizieren der zumindest einen Grafikkodierung an der Behandlungsstation und/oder an einem Teil, das mit der Behandlungsstation zusammenwirkt,
Einlesen (805) einer Patientenidentität eines Patienten, der der Behandlungsstation zugeordnet ist, an der Behandlungsstation,
Übermitteln (805) der Patientenidentität und des zumindest einen Identifikationsmerkmals an eine Zuordnungseinheit (815),
Herstellen einer Zuordnung der Patientenidentität zu dem zumindest einem Identifikationsmerkmal an der Zuordnungseinheit, die eine Identitätspaarung darstellt, Herstellen einer Zuordnung weiterer Datensätze zu der Identitätspaarung, wobei die Datensätze umfassen:
Informationen, die patienten-individuelle therapeutische oder medizinische und/oder physiologische Daten betreffen,
Daten, die das Dialysegerät, dessen Ausrüstung oder die Dialysestation, zu der das Dialysegerät gehört betreffen,
Einlesen (806) der zumindest einen Grafikkodierung an der Behandlungsstation und/oder an einem Teil, das mit der Behandlungsstation zusammenwirkt mit einem mobilen Computer (814),
Dekodieren (807) der zumindest einen Grafikkodierung im mobilen Computer und dadurch Gewinnung des zumindest einen Identifikationsmerkmals,
Übertragen des zumindest einen Identifikationsmerkmals an die Zuordnungseinheit mittels Datenfernübertragung zusammen mit einem Identifikationsmerkmal des mobilen Computers;
Überprüfen der Autorisierung des mobilen Computers in der Zuordnungseinheit anhand des Identifikationsmerkmals des mobilen Computers dahingehend, ob der mobilen Computer zum Empfang von Datenpaketen autorisiert ist,
Senden von Datenpaketen von der Zuordnungseinheit an den mobilen Computer in Abhängigkeit der Überprüfung, wobei die Datenpakete die Identitätspaarung und die der Identitätspaarung zugeordnete Datensätze umfassen.

2. Verfahren nach Anspruch 1, wobei zumindest ein Teil der Grafikkodierung unter Verwendung eines personenspezifischen oder personengruppenspezifischen Schlüssels verschlüsselt wird.

3. Verfahren nach einem der vorhergehenden Ansprüche, wobei die applizierte Grafikkodierung ein Aufkleber ist.

4. Verfahren nach einem der vorhergehenden Ansprüche, wobei die applizierte Grafikkodierung ein Strichcode oder ein zweidimensionaler Code, insbesondere ein QR-Code ist.

5. Verfahren nach einem der vorhergehenden Ansprüche, wobei die Daten, die die Dialysestation betreffen, die Gesamtsituation der Dialysestation beinhalten, wobei die Gesamtsituation **gekennzeichnet ist durch** die Information über die Belegung der Dialysestation, die Identitäten der Patienten, die in der Dialysestation behandelt werden und einen Behandlungsstatus für jeden dieser Patienten.

6. System bestehend aus zumindest einem Dialysegerät (701), einem mobilen Computer (814) und einer Zuordnungseinheit (815), wobei der mobile Computer und die Zuordnungseinheit zur Ausführung der Verfahren, die auf sie gerichtet sind, nach den Ansprüchen 1-5 eingerichtet sind.

## Claims

1. A method for establishing an assignment of a patient identity to a treatment station, wherein the treatment station comprises a dialysis machine (701) and all accessory parts necessary for a pending treatment, comprising the steps:
generating (804) at least one graphic code, which in each case encodes an identification feature of the treatment station and/or of a part that cooperates with the treatment station,
applying the at least one graphic code to the treatment station and/or to a part, which cooperates with the treatment station,
entering (805) a patient identity of a patient assigned to the treatment station at the treatment station,
transmitting (805) the patient identity and the at least one identification feature to an allocation unit (815),
establishing an assignment of the patient identity to the at least one identification feature on the allocation unit, which represents a pairing of identities,
establishing an assignment of further data records to the pairing of identities, wherein the data records comprise:
information about patient-individual therapeutic or medical and/or physiological data,
data about the dialysis machine, the equipment thereof or the dialysis station, to which the dialysis machine belongs,
entering (806) the at least one graphic code on the treatment station and/or on a part that cooperates with the treatment station, by means of a mobile computer (814),
decoding (807) the at least one graphic code in the mobile computer and thus obtaining the at least one identification feature,
transmitting the at least one identification feature to the allocation unit by remote data transmission together with an identification feature of the mobile computer;
checking the authorization of the mobile computer in the allocation unit by means of the identification feature of the mobile computer as to whether the mobile computer is authorized to receive data packets,
sending data packets from the allocation unit to the mobile computer depending on the checking, wherein the data packets comprise the pairing of identities and the data records assigned to the pairing of identities.

2. The method according to Claim 1, wherein at least a portion of the graphic code is encrypted using a key which is specific for a given person or for a group of people.

3. The method according to any one of the preceding claims, wherein the applied graphic code is an adhesive label.

4. The method according to any one of the preceding claims, wherein the applied graphic code is a barcode or a two-dimensional code, in particular a QR code.

5. The method according to any one of the preceding claims, wherein the data about the dialysis station include the overall situation of the dialysis station, wherein the overall situation is **characterized by** the information about the occupancy of the dialysis station, the identities of the patients treated in the dialysis station, and a treatment status for each of these patients.

6. A system comprised of at least one dialysis machine (701), a mobile computer (814), and an allocation unit (815), wherein the mobile computer and the allocation unit are equipped to perform the methods directed thereto, according to claims 1 - 5.

## Revendications

1. Procédé d'établissement d'une association d'une identité de patient à une station de traitement, la station de traitement comprenant un appareil de dialyse (701) et tous les éléments accessoires nécessaires pour un traitement imminent, comprenant les étapes suivantes :
génération (804) d'au moins un codage graphique qui code respectivement une caractéristique d'identification de la station de traitement et/ou d'un élément qui coopère avec la station de traitement,
application de l'au moins un codage graphique à la station de traitement et/ou à un élément qui coopère avec la station de traitement,
lecture (805) d'une identité de patient d'un patient qui est associé à la station de traitement au niveau de la station de traitement,
transmission (805) de l'identité de patient et de l'au moins une caractéristique d'identification à une unité d'association (815),
établissement d'une association de l'identité de patient à l'au moins une caractéristique d'identification au niveau de l'unité d'association, qui constitue un appariement identité,
établissement d'une association d'autres jeux de données à l'appariement d'identité, les jeux de données comprenant :
des informations qui concernent des données thérapeutiques ou médicales et/ou physiologiques individuelles du patient,
des données qui concernent l'appareil de dialyse, son équipement ou la station de dialyse à laquelle l'appareil de dialyse appartient,
lecture (806) de l'au moins un codage graphique au niveau de la station de traitement et/ou d'un élément qui coopère avec la station de traitement à l'aide d'un ordinateur mobile (814),
décodage (807) de l'au moins un codage graphique dans l'ordinateur mobile et ainsi obtention de l'au moins une caractéristique d'identification,
transfert de l'au moins une caractéristique d'identification à l'unité d'association au moyen d'un télé-transfert de données avec une caractéristique d'identification de l'ordinateur mobile,
vérification de l'autorisation de l'ordinateur mobile dans l'unité d'association en partant de la caractéristique d'identification de l'ordinateur mobile pour voir si l'ordinateur mobile est autorisé à recevoir des paquets de données,
envoi de paquets de données par l'unité d'association à l'ordinateur mobile en fonction de la vérification, les paquets de données comprenant l'appariement d'identité et les jeux de données associés à l'appariement d'identité.

2. Procédé selon la revendication 1, dans lequel au moins une partie du codage graphique est codée en utilisant une clé spécifique à une personne ou spécifique à un groupe de personnes.

3. Procédé selon une des revendications précédentes, dans lequel le codage graphique appliqué est un autocollant.

4. Procédé selon une des revendications précédentes, dans lequel le codage graphique appliqué est un code barre ou un code bidimensionnel, en particulier un code QR.

5. Procédé selon une des revendications précédentes, dans lequel les données qui concernent la station de dialyse contiennent la situation globale de la station de dialyse, la situation globale étant **caractérisée par** l'information concernant l'occupation de la station de dialyse, les identités des patients qui sont traités par la station de dialyse et un état de traitement pour chacun de ces patients.

6. Système composé d'au moins un appareil de dialyse (701), un ordinateur mobile (814) et une unité d'association (815), l'ordinateur mobile et l'unité d'association étant conçus pour exécuter les procédés vers lesquels ils sont dirigés selon les revendications 1 - 5.
